# EUROPÄISCHE PATENTSCHRIFT

(11) **EP 0 822 836 B1**
(45) Veröffentlichungstag und Bekanntmachung des Hinweises auf die Patenterteilung: **28.05.2003**
(21) Anmeldenummer: 96908039.9
(22) Anmeldetag: 14.03.1996
(51) Int. Cl.: A61K 49/00, A61B 8/00, B01J 13/04

(54) **VERFAHREN ZUR HERSTELLUNG VON POLYMEREN MIKROPARTIKELN, NACH DIESEM VERFAHREN HERGESTELLTE MIKROPARTIKEL SOWIE DEREN VERWENDUNG IN DER MEDIZINISCHEN DIAGNOSTIK**
PROCESS FOR PRODUCING POLYMER MICROPARTICLES, MICROPARTICLES PRODUCED USING SAID PROCESS, AND THE USE OF SUCH PARTICLES IN MEDICAL DIAGNOSTICS
PROCEDE DE FABRICATION DE MICROPARTICULES POLYMERES, LES MICROPARTICULES FABRIQUES SELON CE PROCEDE ET LEUR APPLICATION POUR LES TECHNIQUES DIAGNOSTIQUES MEDICALES

(30) Priorität: 14.03.1995 DE 19510690
(43) Veröffentlichungstag der Anmeldung: 11.02.1998
(73) Patentinhaber: ACTIPAC Biosystems GmbH, 82152 Martinsried (DE)
(72) Erfinder: RÖSSLING, Georg, D-16548 Glienicke (DE); ALBAYRAK, Celàl, D-10999 Berlin (DE); ROTHE, Matthias, D-12099 Berlin (DE)
(74) Vertreter: VOSSIUS & PARTNER
(86) Internationale Anmeldenummer: EP9601107
(87) Internationale Veröffentlichungsnummer: WO96028191

(56) Entgegenhaltungen:
- EP-A- 0 458 745
- DE-A- 4 219 723
- DE-A- 4 232 755

## Beschreibung

Die Erfindung betrifft den in den Patentansprüchen gekennzeichneten Gegenstand, daß heißt ein Verfahren zur Herstellung von gasenthaltenden Mikropartikeln für die Ultraschalldiagnostik, deren Wandmaterial aus Polyestern von α-, β- oder γ-Hydroxycarbonsäuren aufgebaut ist, Partikel die nach diesem Verfahren herstellbar sind, sowie deren Verwendung in der medizinischen Diagnostik.

Die Ültraschalldiagnostik hat in der Medizin wegen der komplikationslosen einfachen Handhabung sehr breite Anwendung gefunden. Ultraschallwellen werden an Grenzflächen von unterschiedlichen Gewebearten reflektiert. Die dabei entstehenden Echosignale werden elektronisch verstärkt und sichtbar gemacht.

Die Darstellung von Blutgefäßen und inneren Organen mittels Ultraschall erlaubt im allgemeinen nicht die Darstellung des darin vorhandenen Blutflusses. Flüssigkeiten, insbesondere Blut, liefern nur dann Ultraschallkontrast, wenn Dichte- und Kompressibilitätsunterschiede zur Umgebung bestehen. Als Kontrastmittel werden in der medizinischen Ultraschalldiagnostik z. B. Gase enthaltende oder Gas produzierende Substanzen verwendet, da der Impedanzunterschied zwischen Gas und umgebendem Blut wesentlich größer ist, als der von Flüssigkeiten oder Festkörpern und Blut [Levine R.A., J. Am. Coll. Cardiol. 3 (1989) 28 ; Machi I.J. CU 11 (1983) 3].

Roelandt et al. [Ultrasound Med. Biol. 8 (1982) 471-492] beschreiben, daß durch periphere Injektionen von Lösungen, die feine Gasblasen enthalten, cardiale Echokontraste erzielt werden können. Diese Gasblasen werden in physiologisch verträglichen Lösungen z.B. durch Schütteln, andere Agitation oder durch Zusatz von Kohlendioxid erhalten. Sie sind jedoch hinsichtlich Anzahl und Größe nicht standardisiert und können nur unzulänglich reproduziert werden. Auch sind sie in der Regel nicht stabilisiert, so daß ihre Lebensdauer gering ist. Ihre mittleren Durchmesser liegen meist über Erythrocytengröße, so daß keine Lungenkapillarpassage mit nachfolgender Kontrastierung von Organen wie linkes Herz, Leber, Niere oder Milz möglich ist. Darüber hinaus eignen sie sich nicht für Quantifizierung, da sich das von ihnen erzeugte Ultraschallecho aus mehreren, nicht voneinander zu trennenden Prozessen wie Blasenentstehung, Koaleszenz und Auflösung zusammensetzt. So ist es z.B. nicht möglich, mit Hilfe dieser Ultraschall-Kontrastmittel über die Messung des Kontrastverlaufs im Myokard, Aussagen über die Transitzeiten zu gewinnen. Hierzu sind Kontrastmittel notwendig, deren Streukörper eine ausreichende Stabilität aufweisen.

In der EP 0 131 540 ist die Stabilisierung der Gasblasen durch Zucker beschrieben. Damit wird zwar die Reproduzierbarkeit und Homogenität des Kontrasteffektes verbessert, eine Lungenpassage überstehen diese Blasen jedoch nicht.

In den EP 0 122 624 und 0 123 235 wird beschrieben, daß der gasblasenstabilisierende Effekt von Zuckern, Zuckeralkoholen und Salzen durch Zusatz von grenzflächenaktiven Substanzen verbessert wird. Eine Lungenkapillargängigkeit und die Möglichkeit zur Darstellung des arteriellen Gefäßschenkels und verschiedener Organe wie Leber oder Milz ist bei diesen Ultraschallkontrastmitteln gegeben. Der Kontrasteffekt ist hierbei jedoch auf das Gefäßlumen beschränkt, da die Bläschen nicht von den Gewebezellen aufgenommen werden.

Keines der beschriebenen Ultraschall-Kontrastmittel verbleibt längere Zeit unverändert im Körper. Eine Organdarstellung mit ausreichender Signalintensität durch selektive Anreicherung nach i. v. Gabe oder Quantifizierung sind mit diesen Mitteln nicht möglich.

Eine Verkapselung von Gasen, wie beispielsweise Luft als Ultraschall-Kontrastmittel wird in der EP 0 224 934 beschrieben. Das hierbei verwendete Wandmaterial besteht aus Protein, insbesondere menschliches Serumalbumin mit den bekannten allergenen Eigenschaften, zu denen durch eine Denatuierung cytoxische Effekte hinzukommen können.

In der Patentschrift EP 0 327 490 werden gasenthaltende Mikropartikel für die Ultraschall-Diagnostik auf der Basis von biologisch abbaubaren, synthetischen Materialien beschrieben. Als bioabbaubare Polymere werden u.a. auch α-, β- oder γ-Hydroxycarbonsäuren offenbart. Diese Mittel weisen zwar eine ausreichende *in vivo* Lebensdauer auf und werden nach intravenöser Applikation intrazellulär im retikuloendothelialem System und damit auch in der Leber oder Milz angereichert, jedoch zeigen die gemäß den Beispielen dieser Anmeldung hergestellten Partikel auf Basis von Hydroxycarbonsäuren nur einen relativ geringen Rückstreukoeffizenten. Die diagnostische Wirksamkeit daraus bereiteter Kontrastmittelpräparationen ist daher nicht in allen Fällen befriedigend.

EP 0 458 745 offenbart ebenfalls gasenthaltende Mikropartikel für die Ultraschall-Diagnostik.

Aufgabe der vorliegenden Erfindung war es daher ein verbessertes Verfahren zur Herstellung von Mikropartikeln auf der Basis von Polyestern von α-, β- oder γ-Hydroxycarbonsäuren bereitszustellen, nach welchem es gelingt Partikel zu erhalten, die einen besseren Rückstreukoeffizienten aufweisen als die Partikel des Standes der Technik.

Die Partikel und daraus bereitete Kontrastmittelpräparationen sollten weiterhin die übrigen an ein modernes Kontrastmittel gestellten Anforderungen erfüllen, wie z.B. ausreichend klein und stabil sein, um ohne nennenswerten Gasverlust und im wesentlichen quantitativ die linke Herzhälfte nach intravenöser Applikation zu erreichen, eine gute Verträglichkeit aufweisen ohne ein allergenes Potential zu besitzen und nicht im wäßrigen Medium verklumpen.

Diese Aufgabe wird durch die vorliegende Erfindung gelöst.

Es wurde gefunden, daß Mikropartikel auf der Basis von Polyestern von α-, β- oder γ-Hydroxycarbonsäuren hergestellt werden können, indem der jeweilige Polyester und gegebenenfalls eine oberflächenaktive Substanz in einem organischen Lösungsmittel oder Lösungsmittelgemischen, von denen mindestens ein Lösungsmittel mit Wasser gut mischbar ist, gelöst wird, anschließend eine flüssige Perfluoro-Verbindung oder Wasser in dieser Lösung dispergiert wird und anschließend diese Dispersion in Wasser, das eine oberflächenaktive Substanz enthält, mittels eines Rührers dispergiert wird, wobei das Lösungsmittel durch Gaseinleirung und Anlegen eines Vakuums entfernt wird. Hierbei fallen Partikel aus, die zunächst noch Wasser bzw. die flüssige Perfluoro-Verbindung enthalten. Anschließend wird die Partikel enthaltende Suspension mit einem geeigneten pharmazeutisch akzeptablen Kyroprotektor vermischt und gefriergetrocknet, wobei die in den Partikeln befindliche Flüssigkeit weitgehend entweicht und nach Belüften des Lyophilisators durch das gewünschte Gas (in der Regel Luft) ersetzt wird. Je nach Trocknungsdauer verbleibt gegebenenfalls eine geringe Menge der Flüssigkeit (Wasser bzw. Perfluoro-Verbindung) als Dampf in den Partikeln.

Als Perfluoro-Verbindung kommen vorzugsweise Perfluoropentan, Perfluorohexan, Perfluoro-1,3-Dimethylcyclohexan, Perfluorocyclohexen, Perfluorodecalin oder Perfluoroether zur Anwendung.

Als Polymere werden in dem erfindungsgemäßen Verfahren eingesetzt: Polyglycolid (PGA) sowie dessen Copolymere mit L-Lactid (PGA/PLLA) oder Polylactid (PLA) sowie dessen Stereocopolymere wie z.B. Poly-L-Lactid (PLLA), Poly-DL-Lactid oder Poly-L-Lactid/DL-Lactid oder Poly-β-hydroxybutyrat (PHBA) sowie dessen Gopolymere mit β-Hydroxyvalerat (PHBA/HVA) oder Poly-β-hydroxypropionat (PHPA) oder Poly-p-dioxanon (PDS) oder Poly-δ-valerolacton oder Poly-ε-caprolacton.
Bei den Copolymeren zwischen Milchsäure (LA) und Glycolsäure (GA) liegt das Molverhältnis bezogen auf die Monomeren im Bereich von 85:15 bis 50:50, vorzugsweise bei 75:25.

Als organische Lösungs- oder Lösungsmittelgemische für die Polymeren werden bevorzugt, Dichlormethan, Aceton, Ethylacetat, Methylacetat, Triacetin, Triethylcitrat, Ethyllactat, Isopropylacetat, Propylformiat, Butylformiat, Ethylformiat und/oder Methyllactat verwendet.

Als oberflächenaktive Substanz (Tensid) kommen Substanzen aus der Gruppe der Poloxamere® oder Poloxamine®, Polyethylenglycol Alkylether, Polysorbate, Saccharoseester (Sisterna The Netherlands), Saccharoseester [Ryoto sugarester, (Tokyo)], Gelatine, Polyvinylpyrrolidon, Fettalkoholpolyglycosid, Chaps (Serva), Chap (Calbiochem), Chapso (Calbiochem), Decyl-β-D-glycopyranosid, Decyi-β-D-maltopyranosid, Dodecyl-β-D-maltopyranosid, Natriumoleat, Polyethylenglykol, Polyvinylalkohol oder deren Gemische zur Anwendung.

Als Gase kommen Luft, Stickstoff, Sauerstoff. Edelgase, Distickstoffoxid. Kohlendioxid, Halogenkohlenwasserstoffe, gesättigte oder ungesättigte gasförmige Kohlenwasserstoffe, Stickstoffdioxid und/oder Ammoniak zur Anwendung.

Ein alternatives Verfahren zur Herstellung von Mikropartikeln auf der Basis von Polyestern von α-, β- oder γ-Hydroxycarbonsäuren, besteht darin, daß (die) gewünschte(n) Polymer(e) und gegebenenfalls eine Aminosäure in mindestens einem organischen Lösungsmittel gelöst wird (werden), diese Lösung über eine Düse in eine Kolonne gesprüht wird, die mit einem überkritischen Gas befüllt ist oder von diesem durchströmt wird, wobei das Lösungsmittel vom überkritischen Gas aufgenommen wird.

Als Polymere bzw. Copolymere kommen in dieser Verfahrensvariante dieselben Substanzen zur Anwendung, wie sie im erstgenannten Herstellungsverfahren genannt wurden. Entsprechendes gilt fiir die Mischungsverhältnisse bei den Copolymeren.

Vorzugsweise wird zu dem jeweils verwendeten gelösten Polymer (Polyester) anstelle des Tensids eine Aminosäure gegeben. Als Aminosäuren kommen vorzugsweise L-Lysin, L-Phenylalanin, L-Tryptophan sowie D,L-Phenylalanin zum Einsatz.

Als Lösungsmittel bzw. Lösungsmittelgemische eignen sich die in der ersten Verfahrensvariante aufgeführten Lösungsmittel.

Die Zugabe einer Perfluorverbindung ist in diesem Falle nicht erforderlich.

Als überkritische Gase werden, Distickstoffoxid, Kohlendioxid, Halogenkohlenwasserstoffe, gesättigte oder ungesättigte Kohlenwasserstoffe, Stickstoffdioxid und/oder Ammoniak verwendet, wobei Kohlendioxid bevorzugt ist. Die überkritischen Gase können gegebenenfalls bis zu 10% Zusätze wie z.B. niedere Alkohole wie z.B. Ethanol, Ester oder Gase wie z.B. Stickstoff enthalten.

Die Größe der resultierenden Partikel kann über Art, Größe und Form der Einspritzdüse, Arbeitsdruck und Temperatur in der Kolonne gesteuert werden. Eine Partikelgröße wie sie für ein intravenös appliziertes Ultraschallkontrastmittel erforderlich ist (< 10 µm) kann unter Verwendung einer Düse mit einem Düsendurchmesser von 0,5 mm und einem Sprühwinkel von 10° bei einem Arbeitsdruck zwischen 90 und 100 bar, vorzugsweise 94-96 bar und einer Temperatur von 36° C realisiert werden.

Ein weiterer Aspekt der Erfindung betrifft Mikropartikel für die Ultraschalldiagnostik herstellbar nach den vorgenannten Verfahren.

Ein weilerer Aspekt der Erfindung betrifft Kontrastmittel für die Ultraschalldiagnostik enthaltend Mikropartikel die nach den genannten Verfahren hergestellt wurden.

Diese Mittel können erhalten werden, indem die getrockneten Mikropartikel in einem pharmazeutisch akzeptablen Suspensionsmedium resuspendiert werden.

Als pharmazeutisch akzeptable Suspensionsmedien kommen beispielsweise Wasser p.i., wäßrige Lösungen eines oder mehrerer anorganischer Salze wie physiologische Elektrolyt-Lösungen und Pufferlösungen, wie z.B. Tyrode, wäßrige Lösungen von Mono- oder Disacchariden wie Glucose oder Lactose, Zuckeralkohole wie Mannit, die gegebenenfalls zusätzlich noch eine oberflächenaktive Substanz, z.B. aus der Gruppe der Polysorbate oder Polysaccharide enthalten Polyvinylpyrrolidon, Polyethylenglykol, Saccharose Mono- und Diester oder Substanzen aus der Gruppe der Poloxamere oder Poloxamine oder deren Gemische und/oder einem physiologisch verträglichen mehrwertigen Alkohol wie Glycerin in Frage. Bevorzugt ist jedoch für Injektionszwecke geeignetes Wasser.

Um die Sicherheit der Applikation zu erhöhen, kann unmittelbar vor Injektion eine Filtration der Suspension durchgeführt werden.

Die nach den erfindungsgemäßen Verfahren hergestellten Mikropartikel auf der Basis von Polyestern von α-, β- oder γ-Hydroxycarbonsäuren bzw. daraus bereitete Kontrastmittel erfüllen alle Anforderungen, die an ein Ultraschallkontrastmittel gestellt werden. Die in den Mitteln enthaltenen Partikel zeichnen sich durch die folgenden Vorteile aus:
- Sie werden schnell in-vivo abgebaut,
- Abbauprodukte sind toxikologisch unbedenklich,
- sie zirkulieren ausreichend lange im Blutkreislauf,
- sie sind in allen Modi der Ultraschalldiagnostik, insbesondere auch bei Modi bei denen nichtlineare Effekte ausgenutzt werden anwendbar,
- sie sind gut verträglich,
- sie zeigen eine einheitliche, steuerbare Größenverwilung,
- sie sind leicht herstellbar,
- die polymeren Partikel weisen eine enge Molekulargewichtsvertellung auf,
- sie sind ausreichend stabil um auch eine Lungenpassage zu überstehen und eignen sich damit auch für die Kontrastierung des linken Herzens und
- sie werden vom retikuloendothelialen System aufgenommen und eignen sich damit auch für die Kontrastierung der Leber und Milz.

Insbesondere zeigen die nach den erfindungsgemäßen Verfahren hergestellten Partikel einen größeren Rückstreukoeffizenten im Vergleich zu den in der EP 0 327 490 offenbarten Partikeln, die ebenfalls aus α-, β-, γ-Hydroxycarbonsäuren aufgebaut sind. Dieses konnte in einem Vergleichsversuch gezeigt werden. So wurde für eine frisch zubereitete Suspension der in der EP 0 327 490 (Beispiel 1) beschriebenen Partikel in Wasser (Konzentration 18,6 mg/ml) nach 5 Minuten ein Rückstreukoeffizent von αₛ = 4 x 10⁻⁴ [dB/cm] bei 5 MHz Sendefrequenz gemessen, wohingegen für eine erfindungsgemäße Präparation (Partikel nach Beispiel 1 suspendiert in Wasser/Konzentration 0,18 mg/ml) unter sonst identischen Bedingungen ein Rückstreukoeffizient von αₛ = 2,7 x 10⁻¹ [dB/cm] gemessen wurde. Berücksichtigt man, daß die Konzentration der erfindungsgemäßen Präparation um den Faktor 100 geringer war als die Vergleichspräparation, so ergibt sich ein Anstieg der Rückstreukoeffizienten um fünf Zehnerpotenzen. Da der bildgebende Effekt direkt vom Rückstreukoeffizienten abhängig ist, stellen die erfindungsgemäß hergestellten Partikel deutlich effektivere Kontrastmittel dar.

Die Bestimmung der Rückstreukoeffizienten erfolgt in einem *in vitro* Versuchsaufbau, bei dem das von einem in einer Küvette befindlichen Kontrastmittel verursachte rückgestreute Signal gemessen wird (siehe auch "Standardisation of the measurement of acoustical parameters of ultrasound contrast agents" First European Symposium on Ultrasound Contrast Imaging, January 25-26, 1996, Rotterdam).

Die nachfolgenden Beispiele dienen der näheren Erläuterung des Erfindungsgegenstandes, ohne ihn auf diese beschränken zu wollen.

### Beispiel 1

3 g Polymer Resomer® RG-502 (Böhringer Ingelheim) werden in 40 ml Methylenchlorid/Aceton (Volumenanteil 50:50) gelöst. 10 ml Perfluoropentan werden mittels Ultraturrax (10.000 Upm) 2 min lang in der Polymerlösung dispergiert. Die entstandene Emulsion (0/0) wird in 400 ml 1%ige Gelatine Lösung, die auf 0°C temperiert ist, mittels eines mechanischen Rührers (Dispermat-FT, VMA-Getzmann GmbH), (1000 Upm) 30 min lang dispergiert. Die Emulsion (0/0/W) wird in einem 2 l Dreihalskolben, versehen mit einem Rührer (300 Upm) überführt, 3 h lang bei 20°C durch N₂-Einleitung, 3 Stunden lang bei 25°C durch Vakuum das Lösungsmittel entfernt. Anschließend wird die Suspension mit einem Kryoprotektor vermischt und gefriergetrocknet.
Das mit Wasser resuspendierte Lyophilisat enthält Mikropartikel (Durchmesser 0,2-8 µm) und zeigt einen hervorragenden *in vitro* Rückstreukoeffizienten [αₛ = 2,7 x 10⁻¹ [dB/cm] bei 5 mHz Senderfrequenz und einer Teilchenzahl von C = 4 x 10⁶]. Die Bestimmung der Partikeldurchmesser erfolgte mit einem LS-130 der Firma Coulter Electronics GmbH, welche eine Kombination aus Lichtstreuung und Frauenhofer-Beugung ausführt, Die Bestimmung Teilchenanzahl erfolgte mit einem Coulter Counter.

### Beispiel 2

Es wird wie in Beispiel 1 verfahren, wobei Perfluoropentan durch Perfluorodecalin ersetzt wird. Das mit dem Wasser resuspendierte Lyophilisat enthält ultraschallaktive Mikropartikel von 0,2 bis 8 µm Durchmesser.

### Beispiel 3

Es wird wie in Beispiel 1 verfahren, wobei Perfluoropentan durch Perfluorohexan ersetzt wird. Das mit 0,9%iger NaCl-Lösung resuspendierte Lyophilisat enthält ultraschallaktive Mikropartikel von 0,2 bis 8 µm Durchmesser.

### Beispiel 4

Es wird wie in Beispiel 1 verfahren, wobei Perfluoropentan durch Perfluoro-1.3-Dimethylcyclohexan ersetzt wird.
Das mit 5%iger Mannitol-Lösung resuspendierte Lyophilisat enthält ultraschallaktive Mikropartikel von 0,2 bis 8 µm Durchmesser.

### Beispiel 5

Es wird wie in Beispiel 1 verfahren, wobei Perfluoropentan durch Perfluoroheptan ersetzt wird. Das mit 5 %iger Mannitol-Lösung resuspendierte Lyophilisat enthält ultraschallaktive Mikropartikel von 0,2 bis 8 um Durchmesser.

### Beispiel 6

Es wird wie in Beispiel 1 verfahren, wobei das Polymer Resomer® RG-502 durch Resomer® RG-503 ersetzt wird. Das mit 0,9%iger NaCl-Lösung resuspendierte Lyophilisat enthält ultraschallaktive Mikropartikel von 0,2 bis 8 µm Durchmesser.

### Beispiel 7

Es wird wie in Beispiel 1 verfahren, wobei das Polymer Resomer® RG-502 durch Resomer® RG-504 ersetzt und in 45 ml Methylenchlorid/Aceton (Volumentanteil 55:45) gelöst wird. Das mit 0,9%iger NaCI-Lösung aufgenommene Lyophilisat enthält ultraschallaktive Mikropartikel von 0,2 bis 8 um Durchmesser.

### Beispiel 8

Es wird wie in Beispiel 1 verfahren, wobei das Polymer Resomer® RG-502 durch Resomer® RG-756 ersetzt und in 50 ml Methylenchlorid/Aceton (Volumentanteil 60:40) gelöst wird. Das mit Wasser resuspendierte Lyophilisat enthält ultraschallaktive Mikropartikel von 0,2 bis 8 µm Durchmesser.

### Beispiel 9

Es wird wie in Beispiel 1 verfahren, wobei das Polymer Resomer® RG-502 durch Resomer® RG-858 ersetzt und in 65 ml Methylenchlorid/Aceton (Volumentanteil 63:37) gelöst wird. Das mit Wasser resuspendierte Lyophilisat enthält ultraschallaktive Mikropartikel von 0,2 bis 8 µm Durchmesser,

### Beispiel 10

Es wird wie in Beispiel 1 verfahren, wobei Perfluoropentan durch Perfluorohexan und das Polymer Resomer® RG-502 durch Resomer® RG-858 ersetzt und in 65 ml Methylenchlorid/Aceton (Volumentanteil 63:37) gelöst wird. Das mit Wasser aufgenommene Lyophilisat enthält ultraschallaktive Mikropartikel von 0,2 bis 8 µm Durchmesser.

### Beispiel 11

Es wird wie in Beispiel 1 verfahren, wobei Perfluoropentan durch Perfluorodecalin und das Polymer Resomer® RG-502 durch Resomer® RG-858 ersetzt und in 65 ml Methylen chlorid/Aceton (Volumentanteil 63:37) gelöst wird. Das mit Wasser aufgenommene Lyophilisat enthält ultraschallaktive Mikropartikel von 0,2 bis 8 µm Durchmesser.

### Beispiel 12

Es wird wie in Beispiel 1 verfahren, wobei das Polymer Resomer® RG-502 durch Resomer® R-208 ersetzt wird. Das mit Wasser resuspendierte Lyophilisat enthält ultraschallaktive Mikropartikel von 0,2 bis 8 µm Durchmesser.

### Beispiel 13

Es wird wie in Beispiel 1 verfahren, wobei Aceton durch Ethylacetat ersetzt wird. Das mit Wasser aufgenommene Lyophilisat enthält ultraschallaktive Mikropartikel von 0.2 bis 8 µm Durchmesser.

### Beispiel 14

Es wird wie in Beispiel 1 verfahren, wobei das Aceton durch Methylacetat ersetzt wird. Das mit Wasser resuspendierte Lyophilisat enthält ultraschallaktive Mikropartikel von 0,2 bis 8 µm Durchmesser.

### Beispiel 15

Es wird wie in Beispiel 1 verfahren, wobei das Polymer Resomer® RG-502 durch Resomer® RG-858 und Aceton durch Ethylacetat ersetzt wird. Das mit Wasser resuspendierte Lyophilisat enthält ultraschallaktive Mikropartikel von 0,2 - 8 µm Durchmesser.

### Beispiel 16

Es wird wie in Beispiel 1 verfahren, wobei das Perfluoropentan durch 10 ml 20%ige Glucose-Lösung ersetzt wird. Das mit Wasser resuspendierte Lyophilisat enthält ultraschallaktive Mikropartikel von 0,2 bis 8 µm Durchmesser.

### Beispiel 17

Es wird wie in Beispiel 1 verfahren, wobei anstelle von 3 g lediglich 2 g Polymer Resomer® RG-502 eingesetzt wird. Das mit 5%iger Mannitol-Lösung aufgenommene Lyophilisat enthält ultraschallaktive Mikropartikel von 0,1 bis 8 µm Durchmesser.

### Beispiel 18

Es wird wie in Beispiel 1 Verfahren, wobei das Polymer Resomer® RG-502 durch 1g PHB/PHN Copolymer (12% PHV, Biopol ICI), Gelatine Lösung durch 200 ml, 1% PVA Lösung (MW 9 - 10 x 10³ Dalton) ersetzt, das Polymer in 30 ml Methylenchlorid/Aceton (Volumenanteil 66 : 34) gelöst wird.
Das mit Wasser resuspendierte Lyophilisat enthält ultraschallaktive Mikropartikel von 0,4 bis 8 µm Durchmesser.

### Beispiel 19

Es wird wie in Beispiel 1 verfahren, wobei das Polymer Resomer® RG 502 durch 1 g PHB/PVB Copolymer (18% PHV, Biopol ICI), Gelatine Lösung durch 200 ml 1% PVA Lösung in 30 ml Methylenchlorid/ Aceton (Volumenanteil 66 : 34) gelöst wird. Das mit dem Wasser resuspendierte Lyophilisat enthält ultraschallaktive Mikropartikel von 0,4 bis 8 µm Durchmesser.

### Beispiel 20

Es wird wie in Beispiel 1 verfahren, wobei das Polymer Resomer® RG 502 durch 1 g Polycaprolactome [inherent vis: 1.26 dl/g in CHCl₃ bei 30 °C (Birmingham Polymers NC, USA], Gelatine durch 200 ml 1% PVA Lösung (MW 9 - 10 kDal) ersetzt, das Polymer in 30 ml Methylenchlorid/ Aceton (Volumentanteil 70 : 30) gelöst wird.
Das mit Wasser resuspendierte Lyophilisat enthält ultraschallaktive Mikropartikel von 0,2 bis 8 µm Durchmesser.

### Beispiel 21

Es wird wie in Beispiel 1 verfahren, wobei Gelatine durch 400 ml, 3% Pluronic® F 68 Lösung ersetzt wird. Das mit 5 % Mannitollösung resuspendierte Lyophilisat enthält ultraschallaktive Mikropartikel von 0,4 bis 8 µm Durchmesser.

### Beispiel 22

Es wird wie in Beispiel 1 verfahren, wobei Gelatine durch 400 ml, 0.5 % Na-Oleat Lösung ersetzt wird.
Das mit 5% Mannitollösung aufgenommene Lyophilisat enthält ultraschallaktive Mikropartikel von 0,5 bis 8 µm Durchmesser.

### Beispiel 23

Es wird wie in Beispiel 1 verfahren, wobei Gelatine durch 400 ml, 1% Octyl-β-D-Glucopyranosid Lösung ersetzt wird.
Das mit Wasser resuspendierte Lyophilisat enthält ultraschallaktive Mikropartikel von 0,1 bis 8 µm Durchmesser.

### Beispiel 24

Es wird wie in Beispiel 1 verfahren, wobei Gelatine Lösung durch 400 ml, 1% Saccharosepalmitat P-1570 (Ryoto Sugarester) xersetzt wird.
Das mit Wasser resuspendierte Lyophilisat enthält ultraschallaktive Mikropartikel von 0,1 bis 7 µm Durchmesser.

### Beispiel 25

Es wird wie in Beispiel 1 verfahren, wobei Gelatine Lösung durch 400 ml, 1% Saccharose Stearat (Ryoto Sugarester) S-1670 versetzt wird.
Das mit Wasser resuspendierte Lyophilisat enthäit ultraschallaktive Mikropartikel von 0,1 bis 7 µm Durchmesser.

### Beispiel 26

Es wird wie in Beispiel 1 verfahren, wobei anstelle von Gelatine Lösung, 400 ml, 1% Saccharosediester, SP 70 (Sisternen, The Netherlands) eingesetzt wird.
Das mit Wasser resuspendierte Lyophilisat enthält ultraschallaktive Mikropartikel von 0,1 bis 7 µm Durchmesser.

### Beispiel 27

Es wird wie in Beispiel 1 verfahren, wobei Methylenchlorid/ Aceton Lösungsgemisch durch 70 ml Ethylacetat ersetzt, anstelle von 10 ml Perfluoropentan 18 ml Perfluropentan eingesetzt wird.
Das mit Wasser resuspendierte Lyophilisat enthält ultraschallaktive Mikropartikel von 0,1 bis 5 µm Durchmesser.

### Beispiel 28

Es wird wie in Beispiel 1 verfahren, wobei Aceton durch Ethylacetat ersetzt wird.
Das mit Wasser aufgenommene Lyophilisat enthält ultraschallaktive Mikropartikel von 0,1 bis 7 µm Durchmesser.

### Beispiel 29

Es wird wie in Beispiel 1 verfahren, wobei Aceton durch Methylacetat ersetzt wird. Das mit Wasser resuspendierte Lyophilisat enthält ultraschallaktive Mikropartikel von 1 bis 7 µm Durchmesser.

### Beispiel 30

Es wird wie in Beispiel 1 verfahren, wobei Aceton durch Methylactat ersetzt wird. Das mit Wasser resuspendierte Lyophilisat enthält ultraschallaktive Mikropartikel von 1 bis 6 µm Durchmesser.

### Beispiel 31

Es wird wie in Beispiel 1 verfahren, wobei Aceton durch Ethylactat ersetzt wird.
Das mit Wasser aufgenommene Lyophilisat enthält ultraschallaktive Mikropartikel von 0,1 bis 6 µm Durchmesser.

### Beispiel 32

Es wird wie in Beispiel 1 verfahren, wobei Aceton durch Triacetin ersetzt wird.
Das mit Wasser resuspendierte Lyophilisat enthält ultraschallaktive Mikropartikel von 1 bis 7 µm Durchmesser.

### Beispiel 33

Es wird wie in Beispiel 1 verfahren, wobei Aceton durch Triethylcitrat ersetzt wird. Das mit Wasser resuspendierte Lyophilisat enthält ultraschallaktive Mikropartikel von 1 bis 7 µm Durchmesser.

### Beispiel 34

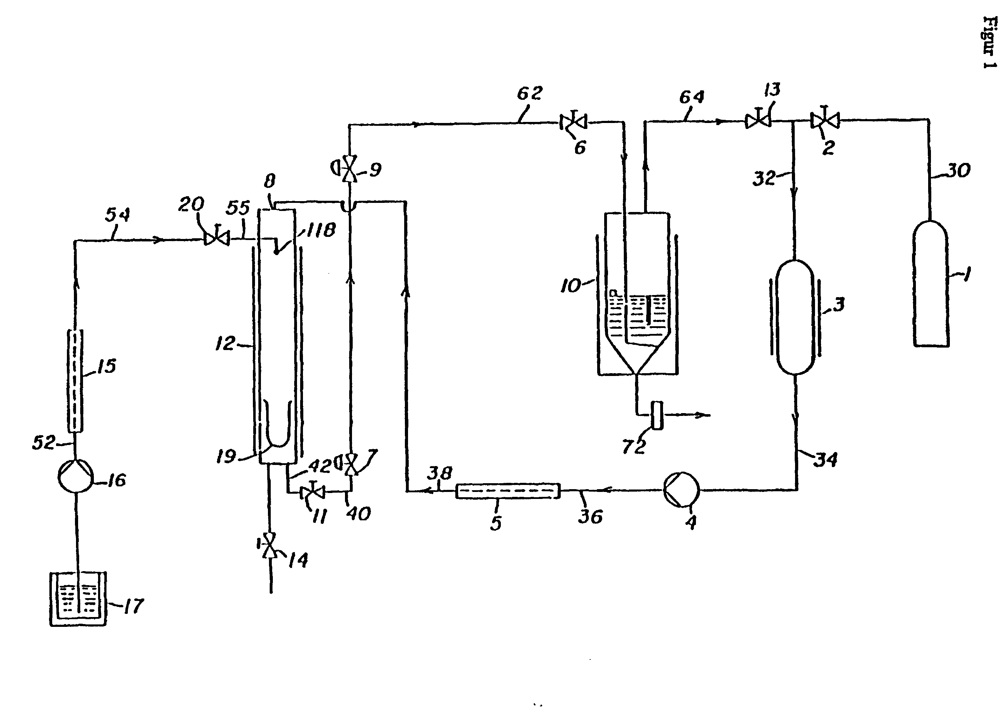

0,375 g L-Lysin (Aldrich) werden in 25 ml Eisessig (Merck) gelöst; 2,5 g Resomer® RG-756 (Böhringer Ingelheim) werden in 75 ml Dichlormethan (Merck) gelöst. Die vereinigten Lösungen werden in einer Apparatur wie in Figur 1 abbgebildet weiter behandelt.

Dazu werden die vereinigten Lösungen zunächst in ein Reservoir (17) gegeben und die Anlage über Ventil (2) und Leitung (30) aus einer Vorratsflasche mit dem Gas befüllt. Mittels einer Kolbenhubpumpe (16) wird die Lösung aus dem Reservoir (17) nach Durchströmen von Leitung (52), einem Wärmetauscher (15), einer Leitung (54), einem Ventil (20) und schließlich Leitung (55), der Düse (118) zugeleitet. Mit einem Druck von 94-96 bar wird die Copolymer enthaltende Lösung durch eine konventionelle Einstoffdüse (118) [Typ Schlick 121 V] in die Kolonne (12) versprüht. wobei gleichzeitig CO₂ im überkritischen Zustand mit 90 bar/36° C und einem Durchsatz von 8,9 kg/h im Gleichstrom, über Einlaß (8), durch die Kolonne geleitet wird. Die Düse hat einen Durchmesser von 0,5 mm, der Sprühwinkel beträgt 10°.

Entsprechend der hohen Affinität des überkritischen CO₂ zum Lösungsmittel wird den primär gebildeten Tröpfchen Lösungsmittel entzogen. Zurück bleiben kugelförmige feste Polymerpartikel.

Die weiteren Schritte dienen im wesentlichen der Reinigung und Rückführung des lösungsmittelbeladenen CO₂, haben aber mit der Herstellung der Partikel nichts mehr zu tun. Die Aufbereitung des CO₂ kann wie folgt geschehen. Das mit dem Lösemittel beladene Gas entströmt durch Leitungen (42) und (40), gesteuert durch 2 Magnetventile (7 und 9), dem Kolonnenende und wird auf 60 bar entspannt. Die Ventile sind so geschaltet, daß die pro Zeiteinheit in die Kolonne einströmende Menge des fluiden Gases unter Aufrechthaltung des Arbeitsdruckes der Kolonne entströmen kann. Das durch die Entspannung auf 60 bar abgekühlte, mit Lösungsmittel beladene CO₂ wird mittels Leitung (62) in den auf 21° C temperierten Separator (10) geleitet, wo sich das Lösungsmittelgemisch infolge der unter diesen Bedingungen stark verminderten Löslichkeiten im CO₂ abscheidet. Das vom Lösungsmittelgemisch befreite CO₂ wird mittels Leitung (64 und 32) durch Druck- und Temperaturerhöhung (3 und 4) erneut in den überkritischen Zustand überführt (90 bar, 36° C) und zur weiteren Trocknung der entstandenen Partikel erneut der Kolonne über Leitung (34), Flüssiggaspumpe (4), Leitung (36), Wärmetauscher (5), Leitung (38) durch Einlaß (8) zugeführt.

Die Entnahme des im Separator (10) abgetrennten Lösungsmittelgemischs erfolgt nach Abtrennung des Separators (10) aus dem Kreislauf durch Ventile (6) und (13) und Entspannung auf Atmosphärendruck über das Ventil (72).

Nachdem die gesamte im Reservoir enthaltene Menge an gelöstem Polymer versprüht wurde (Zeitdauer je nach Druck 20 bis 50 min), wird noch solange CO₂ durch die Kolonne geleitet, bis im Separator (10) keine Lösungsmittelreste mehr zurückgewonnen werden können.

Nach Abschluß des Trocknungsvorganges wird der CO₂-Strom zur Kolonne abgestellt, die Kolonne über die Ventile (11) und (14) auf Atmosphärendruck entspannt und die Partikel am unteren Kolonnenende (19) entnommen.

Es werden ultraschallaktive, gashaltige Mikropartikel mit einem Durchmesser von 5-10 µm erhalten.

### Beispiel 35

0,375 g L-Lysin (Aldrich), 2,5 g Polymer (RG-756) werden in 25 ml Eisessig (Merck) und 75 ml Dichlormethan (Merck) gelöst und solange gerührt, bis eine klare Lösung entstanden ist. Die weitere Umsetzung erfolgt analog zu Beispiel 34.

Es werden ultraschallaktive, gashaltige Mikropartikel mit einem Durchmesser von 5-10 µm erhalten.

### Beispiel 36-41

Die Herstellung weitere Mikropartikel erfolgt analog zu Beispiel 34. Die jeweils verwendeten Polymeren sowie weitere Daten sind in Tabelle 1 zusammengefaßt.

In allen Fällen werden ultraschallaktive, gashaltige Mikropartikel erhalten.
Die erhaltenen ultraschallaktiven, hohlen Mikropartikel sind steril und frei von Lösemittelrückständen, Polymerisationskatalysatoren oder Initiatormolekülen.
Sie besitzen eine gleichmäßige Teilchengrößenverteilung von 5 bis 10 µm.

Die Polymere werden jeweils in 75 ml Dichlormethan (Merck) und 25 ml Eisessig gelöst, die jeweilige Menge Aminosäure wird zugegeben. D,L-Phenylalanin ist geeigneterweise in bis 50 ml Ethanol (Merck) vorzulösen.

**Tabelle 1**

| Versuchsnummer | Polymer bzw. Copolymer | Einwaage Polymer [g] | Aminosäure | Einwaage Aminosäure [g] | Molverhältnis Lactid/Glycolid |
|---|---|---|---|---|---|
| 36 | Poly-(D,L)-lactid | 2,0 | L-Lysin | 0,5 | |
| 37 | Poly-(D,L)-lactid-coglycolid | 1,0 | L-Phenyl-alanin | 0,25 | 75 : 25 |
| 38 | Poly-(D,L)-lactid-coglycolid | 2,0 | D,L-Phenyl-alanin | 0,5 | 75 : 25 |
| 39 | Poly-(D,L)-lactid-coglycolid | 2,2 | L-Tryptophan | 0,55 | 75 : 25 |
| 40 | Poly-L-lactid | 6,0 | L-Lysin | 0,9 | |
| 41 | Poly-L-lactid | 3,0 | L-Lysin | 0,45 | |

## Patentansprüche

1. Verfahren zur Herstellung von gasenthaltenden Mikropartikeln für die Ultaschalldiagnosik, deren Wandmaterial aus Polyestern von α-, β- oder γ-Hydroxycarbonsäuren aufgebaut ist, **dadurch gekennzeichnet, daß**
(i) der jeweilige Polyester und gegebenenfalls eine oberflächenaktive Substanz in einem organischen Lösungsmitteln oder Lösungsmittelgemischen, von denen mindestens ein Lösungsmittel mit Wasser gut mischbar ist, gelöst. wird, anschließend eine perfluorierte Verbindung oder Wasser in dieser Lösung dispergiert wird und anschließend diese Dispersion in Wasser, das eine oberflächenaktive Substanz enthält, mittels eines Rührers dispergiert wird, wobei das Lösungsmittel durch Gaseinleitung und Anlegen eines Vakuums entfernt wird und abschließend die so erhaltene Suspension mit einem geeigneten pharmazeutisch akzeptablen Kryoprotektor vermischt und gefriergetrocknet wird
oder daß
(ii) das (die) gewünschte(n) Polyester und gegebenenfalls eine Aminosäure in mindestens einem organischen Lösungsmittel gelöst wird (werden), diese Lösung über eine Düse in eine Kolonne gespüht wird, die mit einem überkritischen Gas befüllt ist oder von diesem durchströmt wird, wobei das Lösungsmittel vom überkritischen Gas aufgenommen wird.

2. Verfahren nach Anspruch 1, **dadurch gekennzeichnet, daß** das Wandmaterial aus Polyglycolid (PGA) sowie dessen Copolymere mit L-Lactid (PGA/PLLA) oder PolyLaclid (PLA) sowie dessen Stereocopolymere wie z.B. Poly-L-Lactid (PLLA), Poly-DL-Lactid oder L-Lactid/DL-Lactid oder Poly-β-hydroxybutyrat (PHBA) sowie dessen Copolymere mit β-Hydroxyvalerat (PHBA/HVA) oder Poly-β-hydroxypropionat (PHPA) oder Poly-p-dioxanon (PDS) oder Poly-δ-valerolacton oder Poly-ε-caprolacton besteht.

3. Verfahren nach Anspruch 1 oder 2, **dadurch gekennzeichnet, daß** bei Copolymeren aus Milchsäure und Glycolsäure das Mischungsverhältnis im Bereich von 85:15 bis 50:50 liegt.

4. Verfahren nach Anspruch 1, **dadurch gekennzeichnet, daß** als Lösungsmittel Dichlormethan, Aceton, Ethylacetat, Methylacetat, Triacetin, Triethylcitrat, Ethyllactat. Isopropylacetat, Propylformiat, Butylformiat, Ethylformiat und/oder Methyllactat verwendet werden.

5. Verfahren nach Anspruch 1 (i), **dadurch gekennzeichnet, daß** als perfluorierte Verbindungen Perfluoropentan, Perfluorohexan, Perfluoro-1,3-Dimethylcyclohexan, Perfluorocyclohexen, Perfluorodecalin und/oder Perfluoroether verwendet wird.

6. Verfahren nach Anspruch 1 (i), **dadurch gekennzeichnet, daß** als oberflächenaktive Substanz Substanzen aus der Gruppe der Poloxamere oder Poloxamine, Polyethylenglycol Alkylether, Polysorbate, Saccharoseester, Gelatine, Polyvinylpyrolidon, Fettalkoholpolyglycosid, Chaps, Chap, Chapso, Decyl-β-D-glycopyranosid, Decyl-β-D-maltopyranosid, Dodecyl-β-D-maltopyranosid, Natriumoleat, Polyethylenglykol, Polyvinylalkohol oder deren Gemische verwendet werden.

7. Verfahren nach Anspruch 1 (ii), **dadurch gekennzeichnet, daß** als Aminosäure L-Lysin, L-Phenylalanin, L-Tryptophan und/oder D,L-Phenylalanin verwendet wird.

8. Verfahren nach Anspruch 1 (ii), **dadurch gekennzeichnet, daß** als überkritisches Gas Distickstoffoxid, Kohlendioxid, Halogenkohlenwasserstoffe, gesättigte oder ungesättigte gasförmige Kohlenwasserstoffe, Stickstoffdioxid und/oder Ammoniak verwendet wird.

9. Verfahren nach Anspruch 1 (ii) **dadurch gekennzeichnet, daß** die Lösung mit einem Oberdruck von 94-96 bar durch eine konventionelle Einstoffdüse mit einem Düsendurchmesser von 0,5 mm und einem Sprühwinkel von 10° versprüht wird.

10. Mikropartikel fiir die Ultraschalldiagnostik hergestellt nach dem in Anspruch 1 genannten Verfahren.

11. Mikropartikel für die Ultraschalldiagnostik nach Anspruch 10, enthaltend als Gas Luft, Stickstoff, Sauerstoff, Edelgase, Distickstoffoxid, Kohlendioxid, Halogenkohlenwasserstoffe, gesättigte oder ungesättigte gasförmige Kohlenwasserstoffe, Stickstoffdioxid und/oder Ammoniak.

## Claims

1. Method for producing gas-containing microparticles for ultrasonic diagnostics, the wall material of which is formed from polyesters of α-, β- or γ-hydroxycarboxylic acids, **characterised in that**
(i) the respective polyester and if necessary a surface-active substance is dissolved in an organic solvent or solvent mixtures, at least one solvent of which is readily miscible with water, subsequently a perfluorinated compound or water is dispersed in this solution and subsequently this dispersion in water which contains a surface-active substance is dispersed by means of an agitator, the solvent being removed by introduction of gas and application of a vacuum and finally the thus obtained suspension being mixed with a suitable pharmaceutically acceptable cryogenic protector and being freeze-dried
or **in that**
(ii) the desired polyester(s) and if necessary an amino acid is (are) dissolved in at least one organic solvent, this solution being sprayed via a nozzle into a column which is filled with a supercritical gas or flowed through by said gas, the solvent being taken up by the supercritical gas.

2. Method according to claim 1, **characterised in that** the wall material consists of polyglycolide (PGA) and copolymers thereof with L-lactide (PGA/PLLA) or polylactide (PLA) and stereocopolymers thereof, such as e.g. poly-L-lactide (PLLA), poly-DL-lactide or L-lactide/DL-lactide or poly-β-hydroxybutyrate (PHBA) and copolymers thereof with β-hydroxyvalerate (PHBA/HVA) or poly-β-hydroxypropionate (PHPA) or poly-p-dioxanone (PDS) or poly-δ-valerolactone or poly-ε-caprolactone.

3. Method according to claim 1 or 2, **characterised in that**, in the case of copolymers made of lactic acid and glycolic acid, the mixture ratio is in the range of 85 : 15 to 50 : 50.

4. Method according to claim 1, **characterised in that** dichloromethane, acetone, ethyl acetate, methyl acetate, triacetin, triethyl citrate, ethyl lactate, isopropyl acetate, propyl formate, butyl formate, ethyl formate and/or methyl lactate are used as solvent.

5. Method according to claim 1 (i), **characterised in that** perfluoropentane, perfluorohexane, perfluoro-1,3-dimethylcyclohexane, perfluorocyclohexane, perfluorodecaline and/or perfluoroether is used as perfluorinated compound.

6. Method according to claim 1 (i), **characterised in that** substances from the group of poloxamers or poloxamines, polyethylene glycol, alkyl ethers, polysorbates, saccharoesters, gelatine, polyvinylpyrrolidone, fatty alcohol polyglycoside, CHAPS, CHAP, CHAPSO, decyl-β-D-glycopyranoside, decyl-β-D-maltopyranoside, dodecyl-β-D-maltopyranoside, sodium oleate, polyethylene glycol, polyvinyl alcohol or mixtures thereof are used as surface-active substance.

7. Method according to claim 1 (ii), **characterised in that** L-lysin, L-phenylalanine, L-tryptophan and/or D,L-phenylalanine is used as amino acid.

8. Method according to claim 1 (ii), **characterised in that** dinitrogen monoxide, carbon dioxide, halocarbons, saturated or unsaturated gaseous hydrocarbons, nitrogen dioxide and/or ammonia is used as supercritical gas.

9. Method according to claim 1 (ii), **characterised in that** the solution is sprayed with an excess pressure of 94 - 96 bar via a conventional one-piece nozzle with a nozzle diameter of 0.5 mm and a spray angle of 10°.

10. Microparticles for ultrasonic diagnostics produced according to the method mentioned in claim 1.

11. Microparticles for ultrasonic diagnostics according to claim 10, containing air, nitrogen, oxygen, noble gases, dinitrogen monoxide, carbon dioxide, halocarbons, saturated or unsaturated gaseous hydrocarbons, nitrogen dioxide and/or ammonia as gas.

## Revendications

1. Procédé de préparation de microparticules contenant un gaz, pour le diagnostic aux ultra-sons, dont le matériau de paroi est élaboré en polyesters d'acides α-, β- ou γ-hydroxycarboxyliques, **caractérisé en ce que** :
(i) le polyester particulier et le cas échéant, une substance tensioactive sont dissous dans un solvant organique ou des mélanges de solvants, dont au moins un solvant est bien miscible à l'eau, ensuite on disperse un composé perfluoré ou de l'eau dans cette solution, et cette dispersion est dispersée à l'aide d'un agitateur dans de l'eau qui contient une substance tensioactive, où le solvant est éliminé par passage d'un gaz et imposition d'un vide et finalement, on mélange à la suspension ainsi obtenue, un cryoprotecteur pharmaceutiquement acceptable approprié et on lyophilise, ou
(ii) le ou les polyesters souhaités et le cas échéant, un acide aminé sont dissous dans au moins un solvant organique, cette solution est pulvérisée par une buse dans une colonne, qui est remplie d'un gaz supercritique ou est traversée par celui-ci, où le solvant est absorbé par le gaz supercritique.

2. Procédé selon la revendication 1, **caractérisé en ce que** le matériau de paroi consiste en un polyglycolide (PGA) ainsi que ses copolymères avec le L-lactide (PGA/PLLA) ou un polylactide (PLA) ainsi que ses stéréocopolymères comme par exemple, un poly-L-lactide (PLLA), un poly-DL-lactide ou -L-lactide/DL-lactide ou un poly-β-hydroxybutyrate (PHBA) ainsi que ses copolymères avec le β-hydroxyvalérate (PHBA/HVA) ou un poly-β-hydroxypropionate (PHPA) ou une poly-p-dioxanone (PDS) ou une poly-δ-valérolactone ou une poly-ε-caprolactone.

3. Procédé selon la revendication 1 ou 2, **caractérisé en ce que** dans les copolymères d'acide lactique et d'acide glycolique, le rapport de mélange se situe dans l'intervalle allant de 85:15 à 50:50.

4. Procédé selon la revendication 1, **caractérisé en ce que** l'on utilise comme solvant, le dichlorométhane, l'acétone, l'acétate d'éthyle, l'acétate de méthyle, la triacétine, le citrate de triéthyle, le lactate d'éthyle, l'acétate d'isopropyle, le formiate de propyle, le formiate de butyle, le formiate d'éthyle et/ou le lactate de méthyle.

5. Procédé selon la revendication 1(i), **caractérisé en ce que** l'on utilise comme composé perfluoré, le perfluoropentane, le perfluorohexane, le perfluoro-1,3-diméthylcyclohexane, le perfluorocyclohexène, la perfluorodécaline et/ou le perfluoroéther.

6. Procédé selon la revendication 1(i), **caractérisé en ce que** l'on utilise comme substance tensioactive, des substances parmi le groupe des poloxamères ou des poloxamines, un polyéthylèneglycolalkyléther, un polysorbate, un ester de saccharose, la gélatine, la polyvinylpyrolidone, un polyglycoside d'alcool gras, le CHAPS, le CHAP, le CHAPSO, le décyl-β-D-glycopyrannoside, le décyl-β-D-maltopyrannoside, le dodécyl-β-D-maltopyrannoside, l'oléate de sodium, le polyéthylèneglycol, le poly(alcool vinylique) ou leurs mélanges.

7. Procédé selon la revendication 1(ii), **caractérisé en ce que** l'on utilise comme acide aminé, la L-lysine, la L-phénylalanine, le L-tryptophane et/ou la D,L-phénylalanine.

8. Procédé selon la revendication 1(ii), **caractérisé en ce que** l'on utilise comme gaz supercritique, l'hémioxyde d'azote, le dioxyde de carbone, des hydrocarbures halogénés, des hydrocarbures gazeux saturés ou insaturés, le dioxyde d'azote et/ou l'ammoniac.

9. Procédé selon la revendication 1(ii), **caractérisé en ce que** l'on pulvérise la solution avec une surpression de 94-96 bar par une buse classique à un composant, avec un diamètre de buse de 0,5 mm et un angle de pulvérisation de 10°.

10. Microparticules pour diagnostic aux ultra-sons, préparées selon le procédé indiqué à la revendication 1.

11. Microparticules pour diagnostic aux ultra-sons selon la revendication 10, contenant comme gaz, l'air, l'azote, l'oxygène,des gazes rares ,l'hémioxyde d'azote, le dioxyde de carbone, des hydrocarbures halogénés, des hydrocarbures gazeux saturés ou insaturés, le dioxyde d'azote et/ou l'ammoniac.
